# EUROPEAN PATENT APPLICATION

(11) **EP 2 295 029 A1**
(43) Date of publication of application: **16.03.2011**
(21) Application number: 09170172.2
(22) Date of filing: 14.09.2009
(51) Int. Cl.: A61K 8/46, A61K 8/42, A61Q 5/04, A61Q 5/06, A61K 8/23

(54) **Method of chemically modifying the internal region of a hair shaft**

(71) Applicant: The Procter & Gamble Company, Cincinnati, OH 45202 (US)
(72) Inventor: Krause, Thomas, 64297, Darmstadt (DE); Schreiber, Birgit, 64678, Lindenfels (DE); Flohr, Andreas, 61476, Kronberg im Taunus (DE)
(74) Representative: Marollé, Patrick Pierre Pascal

(57) **Abstract**

A method of chemically modifying the internal region of a hair shaft, comprising the steps of: providing and applying to hair a treatment composition comprising from 0.1% to 20% of an ethylenic monomer by weight of the total composition, an initiator and a dermatologically acceptable carrier; and exposing the hair to a relative humidity of at least 70%, within 1 h of applying the treatment composition, and said exposure lasting for 10 to 90 min. Said method provides improved performance, efficacy and/or efficiency.

## Description

### FIELD OF THE INVENTION

The present invention relates to a method of chemically modifying the internal region of a hair shaft, comprising the steps of providing and applying to hair a treatment composition comprising an ethylenic monomer, an initiator and a dermatologically acceptable carrier; and exposing the hair to a relative humidity of at least 70%, within 1 h of applying the treatment composition, and said exposure lasting for 10 to 90 min. Said method provides improved performance, efficacy and/or efficiency.

### BACKGROUND OF THE INVENTION

Hair style retention traditionally has been accomplished by the use of styling products comprising polymers and other components that at least partially coat the hair and act on the surface of hair fibres. Several disadvantages are associated with this approach. Loss of hair style due to passage of time, elevated environmental humidity, excessive motion, etc. may cause a consumer to feel the need to refresh a hair style throughout the day. This often requires application of additional styling product(s). The benefits of traditional styling products also may be diminished when applied to hair to which conditioning agents also have been applied. In addition, application of materials to the surface of hair may compromise the natural feel and appearance of the hair and result in a dull appearance and/or a stiff or otherwise unpleasant feel.

There is a need for consumers with damaged, weak or limp hair to increase the volume of their hair, particularly such consumers that utilise a blow drier. These consumers seek hair styles, treatments and products that allow them to perceive and project to others that they have thicker hair i.e. increased hair volume. Improving hair movement and feel are also important desires of these consumers. Therefore, there is also a desire to enable consumers to be able to concurrently achieve increased volume and better hair feel/mobility.

Methods of, and compositions thereof, chemically modifying the internal region of a hair shaft are known in the art. See for example US2008/0210253A1. Said methods may comprise the steps of applying to the hair a first composition comprising specific monomers and applying to the hair a second composition comprising an initiator. Said methods may result in increased rigidity of the hair shaft after multiple washings and/or wetting of the hair, fewer negative effects of moisture and/or humidity such as frizziness or limpness, and said methods may require application of less styling products and/or may hold the style for longer periods of time.

Whilst said methods provide satisfactory results to the hair shaft, there is a constant need for providing methods resulting in further improved performance, efficacy and/or efficiency. As far as the improved performance is concerned, there is a specific need for providing improved shape retention and style durability. As far as efficiency is concerned, there is a need for improving the penetration of the monomers into the hair shaft and/or improving the polymerization of the monomers, for example increasing the lengths of the polymeric chains within the internal structure of the hair shaft. There is also a need for potentiating the efficacy of the method, for example such that less active(s)/composition(s) are required to achieve a similar performance. In addition, there is also a need for limiting the impact of external factors during the polymerization.

### SUMMARY OF THE INVENTION

The present invention relates to a method of chemically modifying the internal region of a hair shaft, comprising the steps of:
(a) providing and applying to hair a treatment composition comprising from 0.1% to 20% of an ethylenic monomer by weight of the total composition, an initiator and a dermatologically acceptable carrier; and
(b) exposing the hair to a relative humidity of at least 70%, within 1 h of applying the treatment composition, and said exposure lasting for 10 to 90 min.

### BRIEF DESCRIPTION OF THE DRAWINGS

While the specification concludes with claims which particularly point out and distinctly claim the present invention, it is believed that the present invention will be better understood from the following description of preferred embodiments, taken in conjunction with the accompanying drawings, in which like reference numerals identify identical elements and wherein:
FIG. 1 is a bar chart showing percentage add-on (y) for different hair switches following treatments according to the present invention. High relative humidity (dark grey bars) is compared with oven drying (light grey bars).
FIG. 2 is a bar chart showing omega loop force (Ω) at for different hair switches following treatments according to the present invention. High relative humidity (dark grey bars) is compared with oven drying (light grey bars). Untreated switches are shown as a cross-hatched bar.

### DETAILED DESCRIPTION OF THE INVENTION

In all embodiments of the present invention, all percentages are by weight of the total composition, unless specifically stated otherwise. All ratios are weight ratios, unless specifically stated otherwise. All ranges are inclusive and combinable. The number of significant digits conveys neither a limitation on the indicated amounts nor on the accuracy of the measurements.

All numerical amounts are understood to be modified by the word "about" unless otherwise specifically indicated. Unless otherwise indicated, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or byproducts that may be included in commercially available materials, unless otherwise specified.

"Hair," as used herein, means hair on the human head and scalp. "Hair shaft" means an individual hair strand and may be used interchangeably with the term "hair."

"Internal region of the hair shaft," as used herein, means any non-surface portion of the hair shaft, including the inner portion of the cuticle. "Non-surface portion" may be understood to mean that portion of the hair that is not in direct contact with the outside environment.

"Proximal to the scalp," as used herein, means that portion of an extended, or substantially straightened, hair shaft that is closer in distance to the scalp than to the end of the hair. Thus, about 50% of the hair would be considered proximal to the scalp, and about 50% of the hair would be distal to the scalp. "x cm proximal to the scalp" means a distance "x" along the hair, with one endpoint being on or directly adjacent to the scalp, and the second endpoint being measured "x" centimetres along the length of the extended or substantially straightened hair.

"Dermatologically-acceptable carrier," as used herein, means that the compositions or components described are suitable for use in contact with human keratinous tissue without undue toxicity, incompatibility, instability, allergic response, and the like.

"Derivatives," as used herein, includes but is not limited to, amide, ether, ester, amino, carboxyl, acetyl, and/or alcohol derivatives of a given compound.

"Monomer," as used herein, means a discrete, non-polymerised chemical moiety capable of undergoing polymerisation in the presence of an initiator.

"Ethylenic monomer," as used herein, means a chemical species that contains an olefenic carbon-carbon double bond (C=C) and is capable of undergoing polymerization in the presence of an initiator.

"Chemically modify," or grammatical equivalents thereof, as used herein, means that a chemical moiety such as monomer and/or crosslinker and/or polymer, stably affixes to a second chemical moiety, for example, a keratin protein, another component of hair, and/or another monomer or crosslinker.

"Separately packaged," as used herein, means any form of packaging that prevents a color lock composition from coming into physical contact, or admixing, with a second composition. "Separately packaged" may mean that the individual compositions are packaged in separate containers, or alternatively in a single container partitioned such that the compositions are not in physical contact.

"Relative humidity", as used herein, means the amount of water vapour carried in the air. "High relative humidity" herein refers to a relative humidity higher than that at ambient conditions i.e. at least 70%. "Environmental humidity" as used herein relates to relative humidity that the consumer may be exposed to once the method of the present invention has been completed. An example of environmental humidity is that due to weather conditions. "Environmental humidity resistance", as used herein relates to the ability of the hair to better resist negative consequences of environmental humidity.

The present invention relates to method of chemically modifying the internal region of a hair shaft, comprising the steps of:
(a) providing and applying to hair a treatment composition comprising from 0.1% to 20% of an ethylenic monomer by weight of the total composition, an initiator and a dermatologically acceptable carrier; and
(b) exposing the hair to a relative humidity of at least 70%, within 1 h of applying the treatment composition, and said exposure lasting for 10 to 90 min.

The inventors have surprisingly found that by exposing the hair to a relative humidity (also referred to herein as RH) of at least 70%, within 1 h of applying the treatment composition, and said exposure lasting for 10 to 90 min, a method of chemically modifying the internal region of a hair shaft is provided, which results in improved performance.

Indeed, the application of high RH has numerous benefits with regard to this technology. Without being bound by theory, it is believed that exposing the hair to high RH during the chemical modification of the internal region of a hair shaft improves the diffusion of the monomers through the hair and/or increases the penetration of the monomers into the hair shaft. More specifically, it is believed that components of the treatment compositions are maintained in solution for longer and are more mobile. Furthermore, high RH is thought to result in a swollen hair shaft and a more open cuticle.

Exposure to high RH during the chemical modification of the internal region of a hair shaft also limits the impact of external factors during the polymerisation reaction, such as the effects of oxygen. Firstly without being bound by theory, it is believed that oxygen is inhibitory to polymerisation - high oxygen exposure may result in shorter chain lengths of the polymer. Shorter chain length correlates with less optimal benefits to the hair structure and shorter polymer chains will wash out of the hair following a fewer number of washes, compared to longer chain lengths. Therefore, any exclusion of oxygen from the hair is beneficial. During exposure to high RH, the concentration of oxygen in the vicinity of the polymerisation reaction is lower than in the absence of high RH. Secondly, an external surface of the polymerising layer, which is in greater contact with air i.e. more oxygen, may act as a sacrificial layer wherein shorter polymer chain lengths result. However, this sacrificial layer may seal internal polymerisation reactions away from the air and therefore higher oxygen concentrations. Therefore, the internal polymerisation reactions are further protected from oxygen and therefore long polymer chains result. Furthermore, the sacrificial layer may protect the longer chains from wash out.

More generally, it is believed that exposure to high RH potentiates the efficacy of the active(s), such as monomers, initiators, crosslinkers etc, and/or the composition(s), so that a lower quantity of composition(s) and/or lower proportions of active(s) are needed in order to achieve a similar performance and/or a similar quantity of composition(s). Alternatively or additionally, similar proportions of active(s) may achieve improved results, versus a method of chemically modifying the internal region of a hair shaft wherein the hair is not exposed to a relative humidity of at least 70%, within 1 h of applying the treatment composition, and said exposure lasting for 10 to 90 min.

A resultant benefit of the present invention is the provision of a base for highly effective, durable, and optimised hair styling. In particular, benefits may include improved style-ability, enhanced shape retention, increased volume and thickness, longer lasting hold, environmental humidity resistance, increased shine, reduced frizz, dye wash fastness, and style type flexibility (interchangeable between e.g. curled or straightened hair). Furthermore, the internal structure of the hair shaft is improved, and this improvement is retained for longer. The benefits of improved internal structure include greater hair shaft rigidity and strength. Moreover, the surface structure of the hair may benefit. More specifically, the cuticles may form a more regular pattern, which results in increased hair shine. These benefits are superior to what is known in the prior art.

The present invention comprises the provision of a hair treatment composition comprising from 0.1% to 20% of an ethylenic monomer by weight of the total composition, an initiator and a dermatologically acceptable carrier.

The ethylenic monomer has a size suitable to penetrate the hair shaft and is soluble or dispersible in the carrier. The ethylenic monomer may have a molecular weight of about 500 g/mole or less, preferably from about 50 g/mole to about 500 g/mole, more preferably from about 75 g/mole to about 400 g/mole, and even more preferably from about 100 g/mole to about 400 g/mole.

One method of selecting compounds for this technology is selecting from a group of ethylenic monomers, the monomer can be from the family of acrylates, acrylamides, or vinyls. The monomer can penetrate hair to the extent of 5-10% by weight using an aqueous carrier and gravimetric techniques to measure. The monomer may react to about 75% completion within 30 minutes at 35°C in presence of an initiator. In another embodiment the monomer may react from about 90% to about 100% completion within 30 minutes at 35°C in presence of initiator. The final resulting polymer can be water soluble or removable from the surface of hair via normal shampooing. These monomers can be used alone or as co-monomers with other actives to enhance reactivity and solubility.

Suitable ethylenic monomers include the compounds: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, methacryloyl-L-lysine, N-(2-hydroxypropyl)methacrylamide, 2-acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy)ethyl]-trimethylammonium salt, alkylacetamidoacrylate, sulfoalkyl(meth)acrylate and salts, isomers, derivatives and mixtures thereof.

Preferably the ethylenic monomer is a sulfo(meth)acrylate, salts, isomers, derivatives and mixtures thereof. The sulfoalkyl(meth)acrylate compounds of the present invention are understood to be in a non-polymerized form, and do not include polymers of sulfoalkyl(meth)acrylate. More preferably the sulfoalkyl(meth)acrylate compounds is 3-sulfopropylacrylate, CAS #31098-20-1. Sulfopropylacrylate compounds also may be known as sulfopropyl acrylate esters; sulfoalkylpropenoate; acrylic acid propyl ester sulfonates; propenoic acid sulfoalkyl ester; and/or sulfoalkylpropenoic acid ester. The treatment composition may comprise from about 0.1% to about 20%, preferably from about 1% to about 15%, more preferably from about 5% to about 10%, still more preferably from about 0.1% to about 10%, and most preferably from about 10% to about 20% of a sulfopropylacrylate compound, by weight of the total composition.

The ethylenic monomer may also be an alkylacetamidoacrylate compound. "Alkylacetamidoacrylate" as used herein, is understood to include derivatives, salts and/or isomers of alkylacetamidoacrylate. The alkylacetamidoacrylate compounds of the present invention are understood to be in a non-polymerized form, and do not include polymers of alkylacetamidoacrylate. One example of a useful alkylacetamidoacrylate compound is methyl-2-acetamidoacrylate, CAS #35356-70-8. The treatment composition may comprise from about 0.1% to about 20%, preferably from about 1% to about 15%, more preferably from about 5% to about 10%, still more preferably from about 0.1% to about 10%, and most preferably from about 10% to about 20% of a methyl 2-acetamidoacrylate compound, by weight of the total composition, by weight of the total composition.

Alternatively, the ethylenic monomer is a mixture of at least a sulfo(meth)acrylate and an alkylacetamidoacrylate.

The treatment composition also comprises an initiator. Said initiator is useful for promoting binding of an ethylenic monomer and/or crosslinker to the keratin and/or to another monomer. The treatment composition may comprise from about 0.001% to about 5%, preferably from about 0.01% to about 3%, and more preferably from about 0.1% to about 1%, of an initiator, by weight of the total composition. Examples of suitable classes of initiators include, but are not limited to, peroxidisulfates, peroxides, peracids, percarbonate, phosphates, manganates, borates, bis-alkyamidines, sulfites, peroxyesters, bis-cyanocarboxylic acids, alpha-amino acetic acids, and mixtures thereof. Non-limiting examples of suitable initiators include sodium peroxydisulfate, 2,2'-azobis(2-methylpropionamidine)dihydrochloride, 2,2'-azobisisobutyronitrile, benzoyl peroxide, peracetic acid, ammonium cerium(IV) nitrate, hydroxymethanesulfinic acid and mixtures thereof.

The treatment composition may further comprise at least one crosslinker. Alternatively the method may further comprise the step of providing and applying a crosslinker composition comprising a crosslinker. Said crosslinker has a molecular weight of a size suitable to penetrate the hair shaft and a molecular weight of about 500 g/mole or less, preferably from about 100 g/mole to about 500 g/mole, more preferably from about 100 g/mole to about 400 g/mole, and still more preferably from about 200 g/mol to about 400 g/mole. Examples of suitable crosslinkers suitable include, but are not limited to, 1,4-bisacryloylpiperazine, methylenebisacrylamide, ethylenebisacrylamide, divinylbenzene, poly-ethyleneglycol di(meth)acrylate, ethylene glycol di(meth)acrylate, 1,3-butanediol di(meth)acrylate, 1,4-butanediol di(meth)acrylate, neopentyl glycol di(meth)acrylate, Bis[2-(methacryloyloxy)ethyl] phosphate, N,N'-bis(acryloyl)cystamine, N,N-Diallylacryalmide, triallyl cyanurate, 3-(Acryloyloxy)-2-hydroxypropyl methacrylate and mixtures thereof.

The ratio of the weight percentage of the ethylenic monomer to the weight percentage of the crosslinker is preferably from about 50:1 to about 10:1, more preferably from about 40:1 to about 10:1, and still more preferably from about 20:1 to about 10:1.

The treatment composition may further comprise at least one organic or inorganic catalyst. Preferably, the treatment composition comprises from about 0.01% to about 1% of at least one organic or inorganic catalyst, by weight of the total composition. Alternatively the method may further comprise the step of providing and applying a catalyst composition comprising at least one organic or inorganic catalyst. Preferably, the catalyst composition comprises from about 0.01% to about 1% of at least one organic or inorganic catalyst, by weight of the total composition. Non-limiting examples of suitable organic catalysts include 2-pyrolidinoethanol, 1-piperidine-ethanol, 4-methylmorpholine, 2-morpholinoethanol, tetramethylethylenediamine, and mixtures thereof. Non-limiting examples of suitable inorganic catalysts include salts and/or hydrates of cerium, cobalt, manganese, iron, nickel, copper, and mixtures thereof.

The treatment composition may further comprise at least one base. Alternatively, the method may further comprise the step of providing and applying a base composition comprising a base. Said base composition may be packaged separately from the other compositions disclosed herein. Said base composition is useful for adjusting the pH of one, or a combination of any, of the compositions disclosed herein. The base composition may have a pH of above 7.0, preferably of from about 7.0 to about 12.0, more preferably of from about 8.0 to about 12.0, and still more preferably of from about 9.0 to about 11.0. Additionally or alternatively, the base composition has a reserve alkalinity from about 1 to about 40, preferably from about 10 to about 30. Non-limiting examples of classes of suitable bases include, but are not limited to ammonium salts, amines, hydroxides, metasilicates, and mixtures thereof. Non-limiting examples of suitable bases include sodium hydroxide, potassium hydroxide, sodium metasilicate, ammonium hydroxide, ethanolamine, aminomethylpropanol, ammonium carbonate, and mixtures thereof.

The treatment composition may further comprise at least one organic or inorganic salt. Preferably, the treatment composition comprises from about 0.1% to about 10%, and preferably from about 0.5% to about 5%, of at least one organic or inorganic salt, by weight of the total composition. Alternatively, the method may further comprise the step of providing and applying a salt composition. Said salt composition may comprise from about 0.1% to about 10%, and preferably from about 0.5% to about 5%, of at least one organic or inorganic salt, by weight of the total composition. Examples of organic salts include, but are not limited to, salts formed by reacting at least one anion chosen from phosphates, borates, silicates, bicarbonates, carbonates, chlorates, nitrates, halides (including, but not limited to, chlorides), and/or sulfonates, with at least one cation chosen from potassium, sodium, strontium, cadmium, calcium, ammonium (such as tetraalkylammonium and arylammonium), phosphonium, barium, lithium, and/or magnesium. Non-limiting examples of suitable organic salts include sodium monobutyl and dibutyl phosphates and sodium monoethyl and diethyl phosphates. In an embodiment, the salt comprises an inorganic cation. In one embodiment, the inorganic cation is a multivalent cation selected from the group consisting of magnesium, calcium, strontium, barium, copper, zinc, iron, nickel, cobalt, manganese, aluminum, silver, lanthanum, and complexes and mixtures thereof. More preferably the inorganic cation is strontium or aluminium.

The treatment composition may further comprise at least one gelling agent. Preferably, the treatment composition comprises from about 0.1% to about 10%, and preferably from about 0.2% to about 5.0% of a gelling agent, by weight of the total composition. Alternatively, the method may further comprise the step of providing and applying a gelling composition comprising from about 0.1% to about 10%, and preferably from about 0.2% to about 5.0% of a gelling agent, by weight of the total composition. Said gelling agent helps to provide the desired viscosity to the composition(s). Non-limiting examples of suitable optional gelling agents include crosslinked carboxylic acid polymers; unneutralized crosslinked carboxylic acid polymers; unneutralized modified crosslinked carboxylic acid polymers; crosslinked ethylene/maleic anhydride copolymers; unneutralized crosslinked ethylene/maleic anhydride copolymers (e.g., EMA 81 commercially available from Monsanto); unneutralized crosslinked allyl ether/acrylate copolymers (e.g., Salcare™ SC90 commercially available from Allied Colloids); unneutralized crosslinked copolymers of sodium polyacrylate, mineral oil, and PEG-1 trideceth-6 (e.g., Salcare™ SC91 commercially available from Allied Colloids); unneutralized crosslinked copolymers of methyl vinyl ether and maleic anhydride (e.g., Stabileze™ QM-PVM/MA copolymer commercially available from International Specialty Products); hydrophobically modified nonionic cellulose polymers; hydrophobically modified ethoxylate urethane polymers (e.g., Ucare™ Polyphobe Series of alkali swellable polymers commercially available from Union Carbide); and combinations thereof. In this context, the term "unneutralized" means that the optional polymer and copolymer gelling agent materials contain unneutralized acid monomers. Preferred gelling agents include water-soluble unneutralized crosslinked ethylene/maleic anhydride copolymers, water-soluble unneutralized crosslinked carboxylic acid polymers, water-soluble hydrophobically modified nonionic cellulose polymers and surfactant/fatty alcohol gel networks such as those suitable for use in hair conditioning products.

In one embodiment, the treatment composition may be provided as a ready-to-use treatment composition. As used herein, "a ready-to-use composition" means a composition which is sold to the stylist/final user/consumer in a ready-to-use form, i.e. said composition can be applied directly onto hair, without further preparation and/or mixing to be carried out by the stylist/final user/consumer. As an alternative embodiment, the treatment composition may be provided as an extemporaneous treatment composition, i.e. the treatment composition is prepared by mixing at least two different premix compositions together prior to application to hair, preferably within 10 min or less prior to application to hair, more preferably within 1 min or less prior to application to hair. However, because of the reactive chemistry present in the treatment composition, it is preferred to prepare the treatment composition prior to application to hair.

When the treatment composition is prepared prior to application to hair, the method further comprises the step of providing at least a monomer composition comprising an ethylenic monomer - as described herein - and an initiator composition comprising an initiator - as described herein, and mixing both compositions together. Said step precedes the step of applying the extemporaneous treatment composition to hair. The treatment composition may also be provided by mixing, in addition of the monomer composition and the initiator composition, at least one further composition selected from the group consisting of: a salt composition, a gelling composition, a base composition, a catalyst composition, a crosslinker composition or combinations thereof.

In an alternative embodiment, the method of chemically modifying the internal region of a hair shaft, comprises the steps of providing and applying to hair a monomer composition comprising an ethylenic monomer and a dermatologically acceptable carrier; providing and applying to hair an initiator composition comprising an initiator and a dermatologically acceptable carrier; and exposing the hair to a relative humidity of at least 70%, within 1 h of applying the treatment composition, and said exposure lasting for 10 to 90 min. In this embodiment, the steps of applying the monomer composition and the initiator composition may be simultaneous or consecutive, either by applying the monomer composition first and then the initator composition, or by applying the initiator composition first and then the monomer composition.

The compositions of the present invention may comprise from about 60% to about 99.9%, preferably from about 70% to about 95%, and preferably from about 80% to about 90%, of a dermatologically acceptable carrier. Carriers suitable for use with the composition(s) of the present invention include, for example, those used in the formulation of hair sprays, mousses, tonics, gels, and leave-on conditioners. The carrier may comprise water; organic oils; silicones such as volatile silicones, amino or non-amino silicone gums or oils, and mixtures thereof; mineral oils; plant oils such as olive oil, castor oil, rapeseed oil, coconut oil, wheatgerm oil, sweet almond oil, avocado oil, macadamia oil, apricot oil, safflower oil, candlenut oil, false flax oil, tamanu oil, lemon oil and mixtures thereof; waxes; and organic compounds such as C₂-C₁₀ alkanes, acetone, methyl ethyl ketone, volatile organic C₁-C₁₂ alcohols, esters of C₁-C₂₀ acids and of C₁-C₈ alcohols such as methyl acetate, butyl acetate, ethyl acetate, and isopropyl myristate, dimethoxyethane, diethoxyethane, C₁₀-C₃₀ fatty alcohols such as lauryl alcohol, cetyl alcohol, stearyl alcohol, and behenyl alcohol; C₁₀-C₃₀ fatty acids such as lauric acid and stearic acid; C₁₀-C₃₀ fatty amides such as lauric diethanolamide; C₁₀-C₃₀ fatty alkyl esters such as C₁₀-C₃₀ fatty alkyl benzoates; hydroxypropylcellulose, and mixtures thereof. In one embodiment, the carrier comprises water, fatty alcohols, volatile organic alcohols, and mixtures thereof.

The present invention also comprises the step of exposing the hair to a relative humidity of at least 70%, within 1 h of applying the treatment composition, and said exposure lasting for 10 to 90 min. Exposure to high RH during the method of chemically modifying the internal region of a hair shaft has numerous benefits, as discussed above. For example, it limits the impact of external factors during the polymerisation reaction, such as the effects of oxygen. Furthermore, it improves the diffusion of the monomers through the hair and increases the penetration of the monomers into the hair shaft. Moreover, it is believed that components of the treatment compositions are maintained in solution for longer and are more mobile. Additionally, high RH is thought to result in a swollen hair shaft and a more open cuticle.

The hair is exposed to the relative humidity of at least 70%, preferably of at least 85%, more preferably at least 90%, still more preferably at least 95%.

The hair is exposed to the relative humidity for at least 10 min to 90 min, preferably for 20 to 60 min, more preferably 30 to 60 min.

The hair is exposed to the relative humidity within 1 h of applying the treatment composition, preferably within 30 min of applying the treatment composition, more preferably within 20 min of applying the treatment composition.

The hair is exposed to the relative humidity at a preferred temperature of 20°C to 50°C, more preferably from 35°C to 45°C.

The hair is exposed to the relative humidity using a means for covering the hair to reduce the exposure of the hair to oxygen and/or reduce the evaporation of water from the hair. The hair may be covered with an implement such as a shower cap.

In a particularly preferred embodiment of the method, the hair is exposed to the relative humidity by using a device in the vicinity of the hair, preferably less than 0.5 m from the hair, more preferably 1 cm to 30 cm from the hair. Preferably, the device is a water vapour-imparting device, more preferably wherein water vapour-imparting device is an electronic hood-shaped device. Indeed, the inventors have surprisingly found that using such device is particularly suitable for improving the performance, efficacy and/or efficiency of the method according to the present invention. Suitable devices may include: Hairspa ION, by Wella, Darmstadt, Germany; Electronic Master Ionic Action, by Müster; Blitz Super Electronic Ozono, by Ceriotti; Beautivap Digital Ozon and Ozono Energy, by Artem; Mega Ozono, by MediaLine; Mod. 370, by bmp; Steam Machine, by REM; Micro Mist (SD200NIW) and Belmaster (BM-975), by Takara Belmont. A suitable device is described in EP1871194.

The method may further comprise the step of providing and applying to hair a reducing composition comprising a reducing agent and dermatologically acceptable carrier - as described herein. Said step precedes the step of providing and applying a treatment composition. Said reduction composition comprises an agent useful for penetrating the hair shaft and reducing disulfide bonds. The reducing composition comprises from about 1% to about 12%, preferably from about 4% to about 10%, and more preferably from about 8% to about 10%, of a reducing agent, by weight of the total composition. Examples of suitable reducing agents include, but are not limited to, sodium thioglycolate, anhydrous sodium thiosulfate, powdered sodium metabisulfite, thiourea, ammonium sulfite, thioglycolic acid, thiolactic acid, ammonium thiolactate, glyceryl monothioglycolate, ammonium thioglycolate, thioglycerol, 2,5-dihydroxybenzoic acid, diammonium dithioglycolate, strontium thioglycolate, calcium thioglycolate, zinc formosulfoxylate, isooctyl thioglycolate, *D*/*L*-cysteine, monoethanolamine thioglycolate, phosphines, and mixtures thereof.

The method may further comprise the step of providing and applying to hair a finishing composition. Said step follows the exposure of the hair to the high relative humidity. Said finishing composition comprises an oxidising agent and a dermatologically acceptable carrier - as described herein. The oxidising agent may include hydrogen peroxide (about 0.1% to about 3% by weight) and buffered at low pH, and is used to re-oxidise the disulfide bonds and neutralise alkalinity in the hair.

The method may further comprises the step of providing and applying to hair a hair care and/or hair styling composition. Additionally or alternatively the step may comprise providing and utilising an implement for applying styling effects to the hair. Said step(s) follows the exposure of the hair to the high relative humidity.

The hair care and/or hair styling composition may comprise a hair care and/or hair styling active and a dermatologically acceptable carrier - as described herein. Said hair care and/or hair styling active may be selected from the group consisting of hair fixing polymers; conditioning agents, particularly cationic polymers; cleansing agents, particularly surfactants; thickeners; glossing agents; shine-imparting agents; dyes or colour-imparting agents; glitter or coloured particles; silicones; and mixtures thereof. The hair care and/or styling composition may be selected from the group consisting of waxes; gels; hair sprays; mousses; conditioning compositions, particularly leave-in conditioning compositions; finishing sprays; glossing or shine-imparting products; and mixtures thereof.

The implement may be selected from the group consisting of blow dryers; flat irons, combs, brushes, curlers, curling tongs, electrical curling devices, foils, scissors, clips, hair bands, and mixtures thereof.

When the method according to the present invention comprises the steps of subsequently applying at least two compositions onto hair, said method may further comprise the intermediate step of rinsing the first composition by using a rinsing composition and before applying the second composition. The rinsing step may last from 10 sec to 15 min. The rinsing composition may be water. For example, when said method comprises the steps of providing and applying a reducing composition and then a treatment composition to hair, said method may further comprise the intermediate step of rinsing the reducing composition. Likewise when said method comprises the steps of providing and applying a treatment composition and then a finishing composition and/or a hair care and/or hair styling composition to hair, said method may further comprise the intermediate step of rinsing the treatment composition.

Likewise, said method may comprise the intermediate step of drying the hair, preferably by using a towel.

Any of the composition(s) described herein may further comprise one or more optional components known or otherwise effective for use in hair care or personal care products, provided that the optional components are physically and chemically compatible with the essential components described herein, or do not otherwise unduly impair product stability, aesthetics, or performance. Non-limiting examples of such optional components are disclosed in International Cosmetic Ingredient Dictionary, Ninth Edition, 2002, and CTFA Cosmetic Ingredient Handbook, Tenth Edition, 2004, both of which are incorporated by reference herein in their entirety. Some non-limiting examples of such optional components are disclosed below, and include plasticizers, surfactants (which may be anionic, cationic, amphoteric or nonionic), neutralizing agents, propellants, hair conditioning agents (e.g., silicone fluids, fatty esters, fatty alcohols, long chain hydrocarbons, cationic surfactants, etc.), emollients, lubricants and penetrants such as various lanolin compounds, vitamins, proteins, preservatives, dyes, tints, bleaches, reducing agents and other colorants, sunscreens, thickening agents (e.g., polymeric thickeners, such as xanthan gum), physiologically active compounds for treating the hair or skin (e.g., anti-dandruff actives, hair growth actives), non-polymeric thickeners including clays, and perfume.

The present invention may further comprise a kit comprising at least two compositions as described herein, particularly when it is provided at least a monomer composition and an initiator composition so that to prepare the extemporaneous composition prior to application to hair.

"Kit," as used herein, means a packaging unit comprising a monomer composition and a separately packaged initiator composition, as described herein. The kit may further comprise at least one composition selected from a salt composition, a gelling composition, a base composition, a catalyst composition, a crosslinker composition. The kit may also comprise a shower cap for exposing the hair to high relative humidity. The monomer composition and the initiator composition may be packaged in separate containers within the kit, and alternatively may be packaged in a single container which is capable of preventing admixing of the two compositions. The packaging may be of a size suitable for a single application, or unit dose, of the treatment composition.

The kit may further comprise at least one additional composition selected from the group consisting of a pH adjustor, a reducing composition, and combinations thereof. In one embodiment, the pH adjustor has a reserve alkalinity and packaged in a container having a volume, such that when the contents of the container are mixed with the first and/or the second composition, the pH of the resulting mixture of compositions is greater than 7.0. "Reserve alkalinity," as used herein, means the relative strength and apparent concentration of the base used to adjust the pH, measured as described in ASTM D 1121, wherein the base of the present invention is substituted for the antifreeze used in the method.

The kit further may comprise at least one additional component selected from the group consisting of a hair care and/or hair styling composition, a shampoo, a conditioner, a neutralizer, a colorant, a styling aid such as a gel, a mousse, a pomade, etc., an implement, an energy delivery device, instructions for complying with a treatment regimen, and combinations thereof. Examples of energy delivery devices include, but are not limited to light sources, including UV, visible light and infrared light, temperature change elements, hair dryers, heaters such as irons and heated curlers, ultrasonic devices, etc.

The kit further may comprise instructions for complying with a hair treatment regimen. The treatment regimen may comprise one or methods of use described herein, and may be directed toward treatment by a professional stylist or toward treatment by a consumer who is not a professionally-trained stylist. The instructions will direct the user to carry out a method of chemically modifying the internal region of a hair shaft, comprising the steps of: providing and applying to hair a treatment composition comprising from 0.1% to 20% of an ethylenic monomer by weight of the total composition, an initiator and a dermatologically acceptable carrier; and exposing the hair to a relative humidity of at least 70%, within 1 h of applying the treatment composition, and said exposure lasting for 10 to 90 min.

The present invention may further comprise an article of commerce comprising one or more of the compositions, as described herein, and a communication pertaining to the composition(s). The communication may be printed material attached directly or indirectly to packaging, for example to a kit that contains the compositions. Alternatively, the communication may be placed directly or indirectly near at least one composition. Alternatively, the communication may be an electronic or a broadcast message that is associated with the applicator and/or the composition. The communication may comprise images comparing the appearance of a person prior to use of the compositions to the appearance of the same person after use of the composition.

In a particularly preferred embodiment, the present invention relates to a method of chemically modifying the internal region of a hair shaft, comprising the subsequent steps of:
(a) optionally providing and applying to hair a reducing composition, and then rinsing the hair;
(b) providing at least one monomer composition comprising an ethylenic monomer and an initiator composition comprising an initiator, and optionally one additional composition selected from a salt composition, a gelling composition, a base composition, a catalyst composition, a crosslinker composition;
(c) mixing the compositions of step (b) to obtain a treatment composition comprising from 0.1% to 20% of an ethylenic monomer by weight of the total composition, an initiator and a dermatologically acceptable carrier;
(d) within 10 min of step (c), applying to hair said treatment composition;
(e) exposing the hair to a relative humidity of at least 70%, within 1 h of applying the treatment composition, and said exposure lasting for 10 to 90 min, particularly by using a water vapour-imparting device;
(f) rinsing said treatment composition from the hair;
(g) providing and applying to hair a finishing composition comprising an oxidising agent and/or a hair care and/or hair styling composition.

### Examples

The following examples further describe and demonstrate the preferred embodiments within the scope of the present invention. The examples are given solely for the purpose of illustration, and are not to be construed as limitations of the present invention since many variations thereof are possible without departing from its scope.

Hair switches (flat, 2.5 cm wide and 22 cm long) of different colours were treated with the following compositions and then exposed to high RH or control conditions as described hereinafter. For all switches, same amount of composition was added to each switch.

First the switches were treated with a reducing composition, as detailed in Table I, for 10 minutes at 50°C. The reducing compositions comprise either a low concentration (RC low) or a high concentration (RC High) of reducing agent.

**Table I: Reducing Composition**

| **Component** | **Amount (%)** | |
|---|---|---|
| | **RC Low** | **RC High** |
| Demineralised water | Qsp | Qsp |
| Monoethanolamine (85%) | 1.30 | 1.30 |
| 1,2-propylene glycol (double distilled) | 3.00 | 3.00 |
| Hydroxyethylcellulose ¹ | 1.00 | 1.00 |
| Polyoxyethylene (10) coconut alcohol ether (coceth-10) ² | 1.00 | 1.00 |
| PEG-35 castor oil ³ | 1.00 | 1.00 |
| Fragrance | 0.60 | 0.60 |
| Monoethanolamine thioglycolate (67%) | 4.40 | 11.80 |
| Polyquaternium-6 | 0.75 | 0.75 |
| Total | 100.00 | 100.00 |

| | | |
|---|---|---|
| ¹Natrosol 250 HHX from Hercules-Aqualon. ²Genapol C-100 from Clariant. ³Cremophor EL from BASF Corporation. | | |

The switches were then rinsed with water, towel dried, and then treated with a treatment composition obtained by mixing the monomer composition of Table IIa, a salt composition (either Table IIb or IIc) and an initiator composition (Table IId). The relative proportions of the monomer composition, salt composition and initiator composition used are 32.5 g (monomer composition), 31.8 g (salt composition) and 0.65 g (initiator composition), respectively.

**Table IIa: Monomer Composition**

| **Component** | **Amount (%)** |
|---|---|
| Demineralised water | Qsp |
| Cellulose ⁴ | 2.50 |
| Hydroxyethylcellulose ⁵ | 0.30 |
| Disodium EDTA ⁶ | 0.10 |
| Sodium benzoate | 0.25 |
| Sulphuric acid (0.1 N) | 0.40 |
| PEG-35 castor oil ³ | 0.70 |
| Polyoxyethylene (10) coconut alcohol ether (coceth-10) ² | 0.70 |
| Fragrance | 0.30 |
| Phenoxethol | 0.50 |
| 3-sulfopropyl acrylate | 16.00 |
| Total | 100.00 |

| | |
|---|---|
| ⁴Cellulose KC from Nippon Paper Industries, Co., Ltd; ⁵Cellosize HEC QP 4400 from Amerchol; ⁶Rexat and ³Cremophor EL from BASF Corporation; ²Genapol C-100 from Clariant | |

**Table IIb: Salt Composition**

| **Component** | **Amount (%)** |
|---|---|
| Demineralised water | Qsp |
| Strontium Chloride Hexahydrate ⁷ Aluminumsulfate 14-hydrate | 18.77 |
| Ammonia 25% | 3.79 |
| Total | 100.00 |

| | |
|---|---|
| ⁷ from Aldrich; ⁸ from Aldrich | |

**Table IIc: Salt Composition**

| **Component** | **Amount (%)** |
|---|---|
| Demineralised water | Qsp |
| Aluminumsulfate 14-hydrate ⁹ | 27.25 |
| Total | 100.00 |

| | |
|---|---|
| ⁹ from Aldrich | |

**Table IId: Initiator Composition**

| **Component** | **Amount (%)** |
|---|---|
| Sodiumperoxodisulfate¹⁰ | 100.00 |
| Total | 100.00 |

| | |
|---|---|
| ¹⁰ from Aldrich | |

Following exposure to high relative humidity or control conditions (oven drying), the switches were then rinsed with water, towel dried, and then treated with 100 g of a finishing composition of Table III.

**Table III: Finishing Composition**

| **Component** | **Amount (%)** |
|---|---|
| Demineralised water | Qsp |
| Cetearyl alcohol ¹¹ | 2.00 |
| Ceteareth-25 ¹² | 0.50 |
| Salicylic acid | 0.10 |
| Cetyltrimethylammonium chloride ¹³ (25%) | 0.50 |
| Polyquaternium-35 (25% in water, benzoic acid) ¹⁴ | 0.60 |
| Tocopheryl disodium phosphate ¹⁵ | 0.08 |
| Phosphoric acid (85%) | 0.10 |
| Hydrogen peroxide (50%) | 4.00 |
| Fragrance | 0.30 |
| Total | 100.00 |

| | |
|---|---|
| ¹¹Lanette O from Cognis; ¹²Cremophor A 25 from BASF Corporation; ¹³ Detex from Cognis; ¹⁴Bella from Evonik; ¹⁵Dinol from Budenheim. | |

### "Add-On" Measurement

The amount of "add-on" i.e. the percentage weight change in weight of the hair following treatment was measured. Before and after treatment, the switches were weighed on an analytical balance and the difference calculated as a percent. For both weighings, the hair was in dry form.

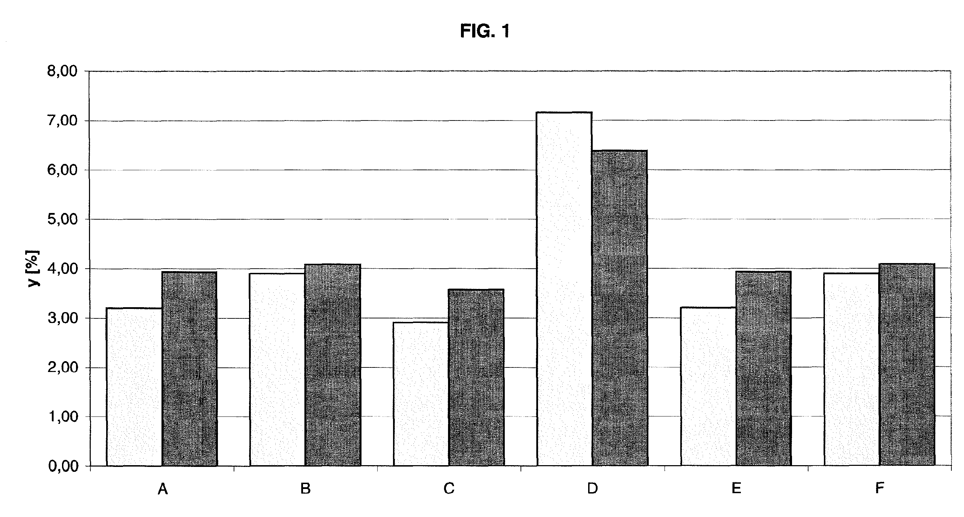

The add-on data is presented in Figure 1. The percentage add-on is shown on the y-axis represented by the letter y. Table IV provides a key for the treatment steps the different switches underwent.

### "Omega Loop" Measurement

The same switches used for the "add on" experiment were then subsequently utilised for omega loop measurement. This procedure involves forming the hair switch lengthwise into the shape of the Greek letter omega (Ω) known as an "omega loop". The hair is clamped at the roots and tips, but the O-shaped part of the hair is not fixed. Compression is then applied on top of the O-part of the omega loop. The amount of force necessary to compress the hair a defined distance is measured.

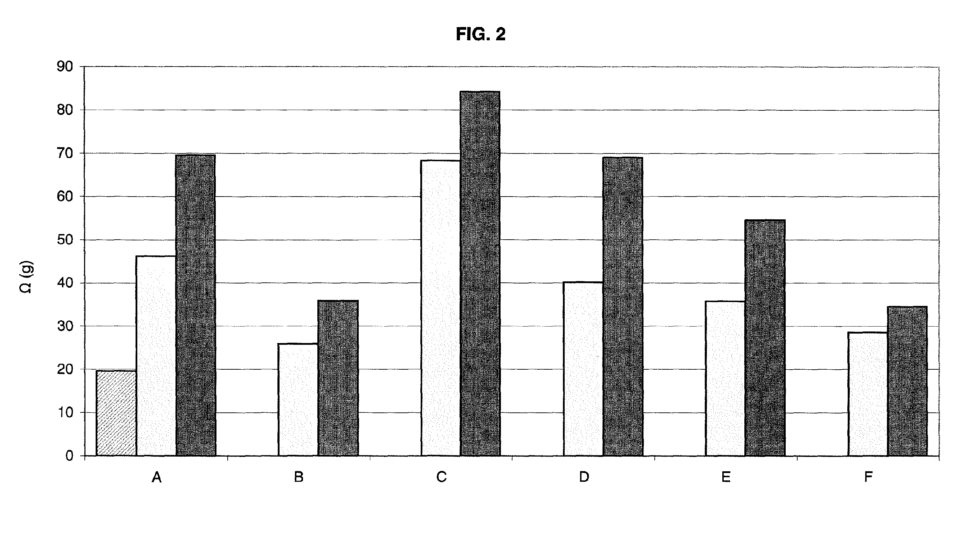

The omega loop data is presented in Figure 2. The force in grams (g) is shown on the y-axis represented by the letter Ω. Table IV provides a key for the treatment steps the different switches underwent.

### Figures

Table IV describes the treatment steps undergone by switches A to F as well as the untreated switches. The data for each set of switches is described in Figures 1 and 2.

**Table IV**

| **Switches** | **Treatment Steps** | |
|---|---|---|
| Untreated | Bleached twice. | |
| A and C | Application of the reducing composition RC low (Table I above); | |
| | Application of a treatment composition comprising: | |
| | | • monomer composition (Table IIa above) |
| | | • salt composition (Table IIc above) |
| | | • initiator composition (Table IId above) |
| | Exposure to high relative humidity or oven dry; | |
| | Application of the finishing composition of Table III. | |
| B and D | Application of the reducing composition RC low (Table I above); | |
| | Application of a treatment composition comprising: | |
| | | • monomer composition (Table IIa above) |
| | | • salt composition (Table IIc above) |
| | | • initiator composition (Table IId above) |
| | Exposure to high relative humidity (30 min) or oven dry (30 min); | |
| | Application of the finishing composition of Table III. | |
| E and F | Application of the reducing composition RC high (Table I above); | |
| | Application of a treatment composition comprising: | |
| | | • monomer composition (Table IIa above) |
| | | • salt composition (Table IIb above) |
| | | • initiator composition (Table IId above) |
| | Exposure to high relative humidity (30 min) or oven dry (30 min); | |
| | Application of the finishing composition of Table III. | |

After treatment according to the present invention, the hair may exhibit one or more benefits, including but not limited to increased shape retention and/or durability, increased appearance of volume, improved shine, improved combability, increased resistance to the effects of humidity, for example, upon the style of the hair and/or upon the condition of the hair. Further examples include all day hold of style, excellent curl definition, increased body and/or fullness, the ability to curl straight hair, and/or the ability to straighten curly hair.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method of chemically modifying the internal region of a hair shaft, comprising the steps of:
(a) providing and applying to hair a treatment composition comprising from 0.1% to 20% of an ethylenic monomer by weight of the total composition, an initiator and a dermatologically acceptable carrier; and
(b) exposing the hair to a relative humidity of at least 70%, within 1 h of applying the treatment composition, and said exposure lasting for 10 to 90 min.

2. The method, according to any preceding claim, wherein the ethylenic monomer has a molecular weight of 500 g/mole or less, preferably from 50 g/mole to 500 g/mole, more preferably from 75 g/mole to 400 g/mole, and even more preferably from 100 g/mole to 400 g/mole.

3. The method, according to any preceding claim, wherein the ethylenic monomer is selected from the compounds: mesaconic acid, 2-pentenoic acid, tiglic acid, tiglic acid esters, furan-3-acrylic acid, 2-acrylamido-2-methyl-1-propanesulfonic acid, maleamic acid, 3-aminocrotonic acid, crotonic acid esters, itaconic anhydride, trimethylsilylacrylate, poly(ethyleneglycol)acrylates, N-vinylacetamide, 2-acetamidoacrylic acid, vinylsulfonic acid, tetrahydrofurfurylacrylate, N-methyl-N-vinylacetamide, vinylpropionate, vinylanisole, vinylcrotonate, methyl 3-hydroxy-2-methylenebutyrate, methacryloyl-L-lysine, N-(2-hydroxypropyl)methacrylamide, 2-acrylamidodiglycolic acid, 2-ethoxyethyl acrylate, 2-butoxyethyl acrylate, N-isopropylmethacryalmide, 2-aminoethyl methacrylate, 2-bromoethyl acrylate, 3-(dimethylamino)propyl acrylate, (3-acrylamidopropyl)trimethyl ammonium salt, [2-(acryloyloxy)ethyl]-trimethylammonium salt, alkylacetamidoacrylate, sulfoalkyl(meth)acrylate and salts, isomers, derivatives and mixtures thereof.

4. The method, according to any preceding claim, wherein the ethylenic monomer is selected from the compounds: sulfo(meth)acrylate, preferably sulfopropylacrylate, alkylacetamidoacrylate, preferably methyl-2-acetamidoacrylate, and mixtures thereof.

5. The method, according to any preceding claim, wherein the initiator is selected from the compounds: sodium peroxydisulfate, 2,2'-azobis(2-methylpropionamidine)dihydrochloride, 2,2'-azobisisobutyronitrile, benzoyl peroxide, peracetic acid, ammonium cerium(IV) nitrate, hydroxymethanesulfinic acid and mixtures thereof.

6. The method, according to any preceding claim, wherein the hair is exposed to the relative humidity of at least 85%, preferably at least 90%, more preferably at least 95%.

7. The method, according to any preceding claim, wherein the hair is exposed to the relative humidity at a temperature of 20°C to 50°C, more preferably from 35°C to 45°C.

8. The method, according to any preceding claim, wherein the hair is exposed to the relative humidity for period of 20 to 60 min, more preferably 30 to 60 min.

9. The method, according to any preceding claim, wherein the hair is exposed to the relative humidity using a means for covering the hair to reduce the exposure of the hair to oxygen and/or reduce the evaporation of water from the hair.

10. The method, according to any preceding claim, wherein the hair is exposed to the relative humidity by using a device in the vicinity of the hair, preferably less than 0.5 m from the hair.

11. The method, according to the preceding claim, wherein the device is a water vapour-imparting device, preferably wherein water vapour-imparting device is an electronic hood-shaped device.

12. The method, according to any preceding claim, further comprising the step of providing and applying a reducing composition comprising a reducing agent, wherein said reducing step precedes the step of providing and applying to hair a treatment composition.

13. The method, according to any preceding claim, further comprises the step of providing and applying a finishing composition comprising an oxidising agent, wherein said finishing step follows the exposure of the hair to the relative humidity.

14. The method, according to any preceding claim, further comprises the step of providing and applying a hair care and/or hair styling composition and/or providing and utilising an implement for applying styling effects to the hair, wherein said step follows the exposure of the hair to the relative humidity.

15. The method, according to the previous claim, wherein the hair care or hair styling composition comprises a hair care or hair styling active selected from the group consisting of hair fixing polymers; conditioning agents, particularly cationic polymers; cleansing agents, particularly surfactants; thickeners; glossing agents; shine-imparting agents; dyes or colour-imparting agents; glitter or coloured particles; silicones; and mixtures thereof.

16. The method, according to any of claims 13 to 15, wherein the implement is selected from the group consisting of blow dryers; flat irons, combs, brushes, curlers, curling tongs, electrical curling devices, foils, scissors, clips, hair bands, and mixtures thereof.
